# EUROPEAN PATENT SPECIFICATION

(11) **EP 3 524 056 B1**
(45) Date of publication and mention of the grant of the patent: **24.02.2021**
(21) Application number: 18156064.0
(22) Date of filing: 09.02.2018
(51) Int. Cl.: A01N 55/10, A01N 25/04, A01N 25/30, A01P 7/02, A01P 7/04, A61K 31/80, A61K 31/695

(54) **COMPOSITIONS FOR USE IN THE TREATMENT OF LICE AND/OR MITES INFESTATIONS ON A HORSE**
ZUSAMMENSETZUNGEN ZUR VERWENDUNG BEI DER BEHANDLUNG VON LÄUSEN UND/ODER MILBENBEFALL BEI PFERDEN
COMPOSITIONS À UTILISER DANS LE TRAITEMENT DES INFESTATIONS DE POUX ET/OU D'ACARIENS SUR UN CHEVAL

(43) Date of publication of application: 14.08.2019
(73) Proprietor: Beaphar B.V., 8101 BX Raalte (NL)
(72) Inventor: Aa, Daan, 8101 BX Raalte (NL)
(74) Representative: V.O.

(56) References cited:
- WO-A1-2008/101131

## Description

### FIELD OF THE INVENTION

The invention disclosed herein relates to fluid compositions for controlling lice and mites on horses.

### BACKGROUND

Controlling lice and mite infestations on horses is a major problem for horse owners. Currently available products for controlling lice and mite infestations on horses are shampoos or lotions that require the horses to be extensively rinsed with water after application of the product. Many horses, however, have long hairs and do not dry up quickly. This is not desired in winter as the (wet) horses may get ill or may develop mud fever. Consequently, many horse-owners do not treat their horses during winter, while lice are abundant during this season.

Another problem is that the currently available products are often based on natural oils and herbs, which are ineffective for controlling lice and mites. Alternative products are often based on insecticides. These products require a prescription from a vet, and are sometimes perceived as less healthy than would be desired for the animals to be treated.

In addition, the fur of a horse is typically greasy, and the fur needs to be degreased to allow a good spread of the active ingredient over the body of the horse.

Furthermore, controlling lice and mite infestations on horses may concern horse-specific insects, such as itchy leg mite (*Chorioptes equi*) and horse lice (*Haematopinus asini* and *Damalinia equi*). Therefore, compositions that work for controlling lice and mites on other animals, e.g. humans, cannot as a rule be expected to also be effective on horses.

In the literature, several approaches to controlling arthropods by using siloxane derivatives are described.

WO01/19190 discloses the use of a composition comprising as active ingredient at least one siloxane, other than solely a linear alkyl or aryl siloxane having a viscosity less than 20,000 centistokes, for the control of arthropods. The document focuses on lice that infest humans (*Pediculus humanus*).

GB1604853 discloses a method of controlling ectoparasites or their ova which comprises applying to a substrate at least one linear siloxane polymer having a viscosity of less than 20,000 centistokes.

WO2015/162395 discloses veterinary compositions comprising dimethicone and cyclomethicone and isopropyl myristate, and the use thereof for the treatment of flea infestation in a pet, preferably a cat or a dog.

WO2012/069785 discloses a fluid composition for the eradication or control of arthropods that is formed at point of use by mixing an emulsion of at least one siloxane compound dispersed in a water continuous phase and a surfactant to thereby demulsify the siloxane compound.

WO2008/087148 discloses the use of a foamable composition for killing arthropods comprising as active ingredient more than 70% by weight of one or more saturated linear or branched hydrocarbons having from 10 to 22 carbon atoms, and as a stabilizing agent between 0.01 and 10% by weight of dimethicone having a viscosity of at least 20,000 centistokes at 25°C.

US2005/0101566 discloses a fluid composition for the eradication or control of arthropods comprising a dispersion of at least one siloxane compound as an emulsion in a water continuous phase and having a dispersed particle size in the range of 1 to 100 nm.

It is desired that compositions are developed that address one or more of the abovementioned problems specifically related to the treatment of lice and mites on horses.

A further background reference is WO 2008/101131 A1, which describes essential-oil compositions comprising Lippia javanica essential oil in combination with at least one essential oil. The Lippia javanica essential-oil compositions are effective for killing and/or repelling ectoparasites and/or pests.

### SUMMARY OF THE INVENTION

In one aspect, the present invention provides a fluid composition comprising:
a) 0.1-20 wt.% of a linear alkyl siloxane having a viscosity in a range of 50 x 10⁻⁶ to 10 x 10⁻³ m²s⁻¹ at 298 K;
b) 5-50 wt.% of an alcohol that is liquid at room temperature;
c) 0.1-30 wt.% of a surfactant;
d) at least 20 wt.% of water;
for use in the treatment of lice and/or mites infestations on a horse.

### DETAILED DESCRIPTION OF THE INVENTION

The invention, in a broad sense, is based on the judicious insight that compositions comprising siloxane, an alcohol, a surfactant, and water, are suitable to control lice and/or mites on horses. Particularly, the compositions are suitable to control horse-specific lice and/or mites. The use of the compositions disclosed herein, does not require rinsing the horse with water after application, nor is it required to use undesirable insecticide. Moreover, the use of the compositions allows a good spread of the active ingredient on the fur and/or skin of the horse.

Without wishing to be bound by theory, it is believed that the evaporation of water from the composition plays a role in their efficacy on horses. Notably, this is believed to increase the concentration of the active ingredient after application, making it more effective. Moreover, it is believed that the addition of a combination of water, alcohol and a surfactant ensures the active ingredient to be evenly spread over the body, skin and/or fur of the horse.

It will be understood that "horse" herein refers to animals within the species of *Equus ferus,* which comprises domesticated horses and Przewalski's horses. Moreover, it will be understood that "fur" herein comprises the mane and tail of a horse.

In one aspect, the composition comprises non-volatile linear alkyl siloxane having a viscosity in a range of 50-10,000 centistokes (*i.e.* 50 x 10⁻⁶ to 10 x 10⁻³ m²s⁻¹). It will be understood that "linear" in this sense relates to compounds in which the siloxanes are linearly coupled. "Alkyl" refers to groups that contain 1-20 carbon atoms, which may be linear, branched or cyclic, and which further may be substituted or unsubstituted, and which may be saturated or (partially) unsaturated. Furthermore, said siloxanes may be modified on one end or on both ends with alkyl, heteroalkyl, aryl, and/or heteroaryl groups or mixtures thereof, each of which may be further substituted. It will be appreciated that "non-volatile" here refers to siloxanes with a boiling point above 300 °C (*i.e.* 573 K) or a viscosity above about 50 centistokes (*i.e.* 50 x 10⁻⁶ m²s⁻¹) at 25 °C (*i.e.* 298 K).

It will be appreciated that the viscosity of the siloxane compounds is given in centistokes, which is a measure of the kinematic viscosity. Kinematic viscosity is the ratio of absolute viscosity to density. For the siloxane compounds used in compositions of the invention, the kinematic viscosity in centistokes is measured at 25 °C (*i.e.* 298 K). Moreover, the kinematic viscosity is determined by methods known to the skilled person, preferably by using a viscometer. Unless stated otherwise, "viscosity" is herein to be understood as "kinematic viscosity".

In some embodiments, the non-volatile linear alkyl siloxane having a viscosity in a range of 50-10,000 centistokes (*i.e.* 50 x 10⁻⁶ to 10 x 10⁻³ m²s^{- 1}) is present in a range from 0.1-20 wt.% relative to the total weight of the composition. Preferably, said siloxane is present in a range from 0.5-15 wt.%, more preferably in a range from 1-10 wt.%, most preferably in a range from 2-7 wt.%. In some embodiments, said siloxane is present at 4 wt.%.

Surprisingly, the composition is effective on horses if said non-volatile linear alkyl siloxanes is the only siloxane present. This is advantageous, in order to keep the number of active ingredients to a minimum. Particularly, it is advantageous that the presence of volatile siloxanes can be avoided.

Without wishing to be bound by theory, it is believed that the presence of non-volatile linear alkyl siloxane having a viscosity in a range of 50-10,000 centistokes (*i.e.* 50 x 10⁻⁶ to 10 x 10⁻³ m²s⁻¹) has a particularly positive effect on the eradication of lice and mites on a horse. It is believed that the siloxane acts mechanically by forming a sticky coat around the louse or mite, which leads to the immobilization of the parasite by blocking their joints. In particular, it is believed that said immobilization causes the louse or mite to all off the horses skin or fur, or at any rate they can be brushed off easily. Furthermore, it is believed that the sticky layer around the louse or mite is formed at a microscopic level that does not lead to the fur or skin of the horse to become sticky.

In a particularly favorable embodiment of the invention, the composition comprises dimethicone or a dimethicone copolyol. Examples of dimethicone copolyols are, but are not limited to, PEG-12 dimethicone, Dimethicone PEG-8 Adipate, Dimethicone PEG-8 Benzoate, Dimethicone PEG-7 Phosphate, Dimethicone PEG-10 Phosphate, Dimethicone PEG/PPG-20/23 Benzoate, Dimethicone PEG/PPG-7/4 Phosphate, Dimethicone PEG/PPG-12/4 Phosphate, PEG-3 Dimethicone, PEG-7 Dimethicone, PEG-8 Dimethicone, PEG-9 Dimethicone, PEG-10 Dimethicone, PEG-14 Dimethicone, PEG-17 Dimethicone, PEG/PPG-3/10 Dimethicone, PEG/PPG-4/12 Dimethicone, PEG/PPG-6/11 Dimethicone, PEG/PPG-8/14 Dimethicone, PEG/PPG-14/4 Dimethicone, PEG/PPG-15/15 Dimethicone, PEG/PPG-16/2 Dimethicone, PEG/PPG-17/18 Dimethicone, PEG/PPG-18/18 Dimethicone, PEG/PPG-19/19 Dimethicone, PEG/PPG-20/6 Dimethicone, PEG/PPG-20/15 Dimethicone, PEG/PPG-20/20 Dimethicone, PEG/PPG-20/23 Dimethicone, PEG/PPG-20/29 Dimethicone, PEG/PPG-22/23 Dimethicone, PEG/PPG-22/24 Dimethicone, PEG/PPG-23/6 Dimethicone, PEG/PPG-25/25 Dimethicone and PEG/PPG-27/27 Dimethicone.

The compositions of the invention further comprise an alcohol that is liquid at room temperature. Without wishing to be bound by theory, it is believed that the alcohol degreases the horse's fur. As a result, it is assumed that alcohol enables a better spread of the active agent.

Typically, said alcohol is present in a range from 5-50 wt.% relative to the total weight of the composition. In more preferred embodiments, the composition comprises said alcohol in a range from 10-30 wt.%, or even more preferably in a range from 15-25 wt.%.

In some embodiments, the alcohol is selected from the group consisting of methyl alcohol, ethyl alcohol, isopropyl alcohol, n-butyl alcohol, sec-butanol, tert-butyl alcohol and isobutyl alcohol. Preferably, the alcohol is ethyl alcohol (ethanol).

Moreover, the compositions of the invention comprise a surfactant. Preferably, the surfactant is nonionic, such as fatty alcohol ethoxylates, alkylphenol ethoxylates, fatty acid ethoxylates, fatty acid esters of glycerol, fatty acid esters of sorbitol (including Span® and Tween®), fatty acid esters of sucrose, and alkyl polyglucosides. In some embodiments, a non-foaming surfactant, of which plenty examples are known to the skilled person, is preferred. In particularly favorable embodiments, the surfactant is selected from the group consisting of polysorbate 20, polysorbate 40, polysorbate 60, polysorbate 80, Span® 20, Span® 60, Span® 65, Span® 80, and Span® 85. It will be understood that polysorbates are also known under their tradename Tween®.

In some embodiments, the composition comprises a surfactant that is present within a range of 0.1-30 wt.% relative to the total weight of the composition. Preferably, said surfactant is present within a range of 1-10 wt.%, more preferably in a range from 3-7 wt.%.

In further embodiments, the compositions of the invention comprises water. It will be understood that this could be water from different sources, such as tap water or ultrapure water. Preferably, demineralized water is used.

In a broad sense, the compositions of the invention comprise at least 20 wt.% of water relative to the total weight of the composition. In preferred embodiments, the water content is at least 40 wt.%. More preferably, the water content is in a range from 50-80 wt.%, most preferably in a range from 60-75 wt.%.

In another aspect, the compositions of the invention may comprise at least one odorant. Non-limiting examples of such odorants are peppermint extract, pine oil, and eucalyptus oil. The skilled person will be able to find more examples of odorants or perfumes in for instance the cosmetic database CoSing. In some embodiments, said odorant is present in a range from 0-5 wt.% with respect to the total weight of the composition. Preferably, said odorant is present in a range from 0.25-2 wt.%, more preferably in a range from 0.5-1 wt.%.

In a still further embodiment, the compositions of the invention comprise at least one skin care agent or skin conditioning agent suitable for the application onto the fur and/or skin of a horse. A non-limiting example of such a skin care agent is aloe vera and extracts thereof. The skilled person will be able to find more examples of skin care agents in for instance the cosmetic database CoSing. Typically, the skin care agent is present in a range from 0-5 wt.% with respect to the total weight of the composition. Preferably, said skin care agent is present in a range from 0.25-2 wt.%, more preferably in a range from 0.5-1 wt.%.

In some embodiments, the composition of the invention is liquid at room temperature. In preferred embodiments, the composition of the invention is also liquid at lower temperatures, for example -30 °C (*i.e.* 243 K), preferably at -20 °C (*i.e.* 253 K), more preferably at -10 °C (*i.e.* 263 K).

The compositions of the invention are prepared by mixing the desired compounds and fluids at appropriate concentrations.

The invention is hereinafter illustrated with reference to the following, non-limiting, examples.

### Example 1

Two Shetland ponies (a mare and a gelding) were diagnosed by a vet to suffer from a lice infestation after the owner discovered bald patches in their fur. The vet advised to treat the ponies with a shampoo.

Instead of with a shampoo, however, the ponies were treated with a composition as listed in Table 1.

**Table 1. A composition according to the invention given in International Nomenclature Cosmetic Ingredient (INCI) names.**

| | Component | | | Function |
|---|---|---|---|---|
| A | PEG-12 dimethicone | 4.0 | %w/w | active |
| B | Aqua | 68.6 | %w/w | solvent |
| C | Ethanol | 20.8 | %w/w | solvent |
| D | Polysorbate 80 | 5.0 | %w/w | nonionic surfactant |
| E | Eucalyptus oil | 0.5 | %w/w | Odorant |
| F | Pine oil | 0.25 | %w/w | |
| G | Peppermint extract | 0.25 | %w/w | |
| H | Aloe Barbadensis extract | 0.5 | %w/w | skin conditioning agent |
| I | DIETHYL PHTHALATE | 0.105 | %w/w | Ethanol denaturant |

After the first treatment, the mare was already cured from her lice infestation. One day after applying the composition of the invention, no moving lice were found in her fur or on her skin. The infestation seemed to be more severe for the gelding. One day after applying the composition of the invention, no moving lice were found on large parts of his body. The tail and the mane of the gelding were however still infested. This showed that treatment of a lice infestation is not easy when ponies have a thick winter fur. Two days after the first application of the composition of the invention to the gelding, it was treated for the second time. Now, only the still infested places were treated. Afterwards, the infestation had completely disappeared.

### Example 2

A Frisian horse, a gelding, is diagnosed by a vet with a mild infestation of lice and itchy leg mite. The owners do not wish to treat their horse with an insecticide as that is incompatible with their lifestyle. Therefore, the horse was successfully treated with a composition of the invention as listed above in Table 1. After the first treatment, the lice infestation had disappeared. The itchy leg mite required a second treatment before the associated health complaints disappeared completely.

## Claims

1. A fluid composition comprising:
a) 0.1-20 wt.% of a linear alkyl siloxane having a viscosity in a range of 50 x 10^{- 6} to 10 x 10⁻³ m²s⁻¹ at 298 K;
b) 5-50 wt.% of an alcohol that is liquid at room temperature;
c) 0.1-30 wt.% of a surfactant;
d) at least 20 wt.% of water;
for use in the treatment of lice and/or mites infestations on a horse.

2. A composition for use according to claim 1, wherein the composition does not comprise a volatile siloxane.

3. A composition for use according to claim 1 or 2, wherein the composition further comprises at least one odorant.

4. A composition for use according to any one of the preceding claims, wherein the composition further comprises at least one skin care agent.

5. A composition for use according to any one of the preceding claims, wherein the linear alkyl siloxane is present in an amount of from 1 to 10 wt.%.

6. A composition for use according to any one of the preceding claims, wherein the alcohol is present in an amount of from 10 to 30 wt.%.

7. A composition for use according to any one of the preceding claims, wherein the surfactant is present in an amount of from 1-10 wt.%.

8. A composition for use according to any one of the preceding claims, comprising at least 40 wt.% of water.

9. A composition for use according to any one of the preceding claims, wherein the linear alkyl siloxane is dimethicone or a dimethicone copolyol.

10. A composition for use according to any one of the preceding claims, wherein the alcohol is selected from the group consisting of methyl alcohol, ethyl alcohol, isopropyl alcohol, n-butyl alcohol, sec-butanol, tert-butyl alcohol and isobutyl alcohol.

11. A composition for use according to any one of the preceding claims, wherein the surfactant is a nonionic surfactant.

12. A composition for use according to claim 11, wherein the surfactant is selected from the group consisting of fatty alcohol ethoxylates, alkylphenol ethoxylates, fatty acid ethoxylates, fatty acid esters of glycerol, fatty acid esters of sorbitol, fatty acid esters of sucrose, and alkyl polyglucosides.

13. A composition for use according to claim 12, wherein the surfactant is selected from the group consisting of polysorbate 20, polysorbate 40, polysorbate 60, and polysorbate 80.

14. A composition for use according to any one of the preceding claims, wherein said linear alkyl siloxane is the only siloxane.

15. A composition for use according to any one of the preceding claims, wherein the linear alkyl siloxane is PEG-12 dimethicone.

## Patentansprüche

1. Flüssige Zusammensetzung, umfassend:
a) 0,1-20 Gew.-% eines linearen Alkylsiloxans mit einer Viskosität in einem Bereich von 50 x 10⁻⁶ bis 10 x 10⁻³ m²s⁻¹ bei 298 K;
b) 5-50 Gew.-% eines Alkohols, der bei Raumtemperatur flüssig ist;
c) 0,1-30 Gew.-% eines Tensids;
d) wenigstens 20 Gew.-% Wasser;
zur Verwendung bei der Behandlung von Läuse- und/oder Milbenbefall beim Pferd.

2. Zusammensetzung zur Verwendung nach Anspruch 1, wobei die Zusammensetzung kein flüchtiges Siloxan enthält.

3. Zusammensetzung zur Verwendung nach Anspruch 1 oder 2, wobei die Zusammensetzung außerdem wenigstens einen Geruchsstoff enthält.

4. Zusammensetzung zur Verwendung nach einem der vorangehenden Ansprüche, wobei die Zusammensetzung ferner wenigstens ein Hautpflegemittel umfasst.

5. Zusammensetzung zur Verwendung nach einem der vorhergehenden Ansprüche, wobei das lineare Alkylsiloxan in einer Menge von von 1 bis 10 Gew.-% vorhanden ist.

6. Zusammensetzung zur Verwendung nach einem der vorhergehenden Ansprüche, wobei der Alkohol in einer Menge von von 10 bis 30 Gew.-% vorhanden ist.

7. Zusammensetzung zur Verwendung nach einem der vorhergehenden Ansprüche, wobei das Tensid in einer Menge von von 1-10 Gew.-% vorhanden ist.

8. Zusammensetzung zur Verwendung nach einem der vorangehenden Ansprüche, umfassend wenigstens 40 Gew.-% Wasser.

9. Zusammensetzung zur Verwendung nach einem der vorhergehenden Ansprüche, wobei das lineare Alkylsiloxan Dimethicon oder ein Dimethicon-Copolyol ist.

10. Zusammensetzung zur Verwendung nach einem der vorhergehenden Ansprüche, wobei der Alkohol ausgewählt ist aus der Gruppe bestehend aus Methylalkohol, Ethylalkohol, Isopropylalkohol, n-Butylalkohol, sec-Butanol, tert-Butylalkohol und Isobutylalkohol.

11. Zusammensetzung zur Verwendung nach einem der vorhergehenden Ansprüche, wobei das Tensid ein nichtionisches Tensid ist.

12. Zusammensetzung zur Verwendung nach Anspruch 11, wobei das Tensid ausgewählt ist aus der Gruppe bestehend aus Fettalkoholethoxylaten, Alkylphenolethoxylaten, Fettsäureethoxylaten, Fettsäureestern von Glycerin, Fettsäureestern von Sorbit, Fettsäureestern von Saccharose und Alkylpolyglucosiden.

13. Zusammensetzung zur Verwendung nach Anspruch 12, wobei das Tensid ausgewählt ist aus der Gruppe bestehend aus Polysorbat 20, Polysorbat 40, Polysorbat 60 und Polysorbat 80.

14. Zusammensetzung zur Verwendung nach einem der vorhergehenden Ansprüche, wobei das lineare Alkylsiloxan das einzige Siloxan ist.

15. Zusammensetzung zur Verwendung nach einem der vorhergehenden Ansprüche, wobei das lineare Alkylsiloxan PEG-12-Dimethicon ist.

## Revendications

1. Une composition de fluide comprenant :
a) 0,1-20 % pds de siloxane linéaire d'alkyle ayant une viscosité dans une plage de 50 x 10⁻⁶ à 10 x 10⁻³ m²s⁻¹ à 298 k ;
b) 5-50 % pds d'alcool qui est liquide à température ambiante ;
c) 0,1-30 % pds d'un agent tensio-actif ;
d) Au moins 20 % pds d'eau ;
pour son utilisation pour le traitement des infestations de poux et mites sur le cheval.

2. Une composition pour son utilisation selon la revendication 1, où la composition ne contient pas de siloxanes volatils.

3. Une composition pour son utilisation selon la revendication 1 ou 2, où la composition contient également au moins un agent odorant.

4. Une composition pour son utilisation selon l'une quelconque des revendications précédentes, où la composition contient également au moins un agent de soin de la peau.

5. Une composition pour son utilisation selon l'une quelconque des revendications précédentes, où le siloxane linéaire d'alkyle est présente selon une teneur de 1 à 10 % pds.

6. Une composition pour son utilisation selon l'une quelconque des revendications précédentes, où l'alcool est présent selon une teneur de 10 à 30 % pds.

7. Une composition pour son utilisation selon l'une quelconque des revendications précédentes, où l'agent tensio-actif est présent selon une teneur de 1 à 10 % pds.

8. Une composition pour son utilisation selon l'une quelconque des revendications précédentes, comprenant au moins 40 % pds d'eau.

9. Une composition pour son utilisation selon l'une quelconque des revendications précédentes, où le siloxane linéaire d'alkyle est la diméthicone ou la diméthicone copolyol.

10. Une composition pour son utilisation selon l'une quelconque des revendications précédentes, où l'alcool est sélectionné dans le groupe composé de l'alcool méthylique, de l'alcool éthylique, de l'alcool isopropylique, de l'alcool butylique, du sec-butanol, de l'alcool tert-butylique et de l'alcool isobutylique.

11. Une composition pour son utilisation selon l'une quelconque des revendications précédentes, où l'agent tensio-actif est un surfactant non ionique.

12. Une composition pour son utilisation selon la revendication 11, où l'agent tensio-actif est sélectionné dans le groupe composé des éthoxylates d'alcools gras, des alkylphénols éthoxylés, des éthoxylates d'acide gras, des esters d'acides gras de glycérol, esters d'acides gras de sorbitol, esters d'acides gras de saccharose et des polyglucosides alkyliques.

13. Une composition pour son utilisation selon la revendication 12, où l'agent tensio-actif est sélectionné dans le groupe composé du polysorbate 20, du polysorbate 40, du polysorbate 60 et du polysorbate 80.

14. Une composition pour son utilisation selon l'une quelconque des revendications précédentes, où ledit siloxane linéaire d'alkyle est le seul siloxane.

15. Une composition pour son utilisation selon l'une quelconque des revendications précédentes, où le siloxane linéaire d'alkyle est le PEG-12 diméthicone.
